# EUROPÄISCHE PATENTSCHRIFT

(11) **EP 1 259 229 B1**
(45) Veröffentlichungstag und Bekanntmachung des Hinweises auf die Patenterteilung: **15.06.2005**
(21) Anmeldenummer: 01909743.5
(22) Anmeldetag: 13.02.2001
(51) Int. Cl.: A61K 31/00, A61P 15/00

(54) **VERWENDUNG VON BENZO-THIENO[2,3-D]PYRIMIDINEN MIT PDE V-INHIBITORISCHER WIRKUNG ZUR BEHANDLUNG VON EREKTILER DYSFUNKTION**
USE OF BENZO-THIENO[2,3-d]PYRIMIDINES HAVING PDE V INHIBITING ACTIVITY FOR THE TREATMENT OF ERECTILE DYSFUNCTION
UTILISATION DE BENZO-THIENO[2,3-d]PYRIMIDINES AYANT UNE ACTIVITE INHIBITRICE DE LA PDE V POUR LE TRAITEMENT D'UN DYSFONCTIONNEMENT ERECTILE

(30) Priorität: 03.03.2000 DE 10010612
(43) Veröffentlichungstag der Anmeldung: 27.11.2002
(73) Patentinhaber: MERCK PATENT GmbH, 64293 Darmstadt (DE)
(72) Erfinder: BRÄNDLE, Marian, 72108 Rottenburg (DE); EHRING, Thomas, 42853 Remscheid (DE); WILM, Claudia, 64291 Darmstadt (DE)
(86) Internationale Anmeldenummer: PCT/EP2001/001557
(87) Internationale Veröffentlichungsnummer: WO 2001/064192

(56) Entgegenhaltungen:
- WO-A-00/10542
- WO-A-00/66099
- WO-A-00/78767
- WO-A-98/53819
- WO-A-99/55708
- WO-A-99/66924

## Beschreibung

Die Erfindung betrifft die Verwendung von PDE V - lnhibitoren der Formel I 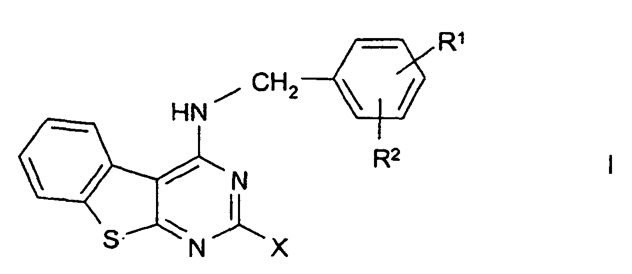 worin
- R¹, R²: jeweils unabhängig voneinander H, A, OA, OH oder Hal,
- R¹ und R²: zusammen auch Alkylen mit 3-5 C-Atomen, -O-CH₂-CH₂-, -CH₂-O-CH₂-, -O-CH₂-O- oder -O-CH₂-CH₂-O-,
- X: einfach durch R⁷ substituiertes R⁴, R⁵ oder R⁶,
- R⁴: lineares oder verzweigtes Alkylen mit 1-10 C-Atomen, worin eine oder zwei CH₂-Gruppen durch -CH=CH-Gruppen ersetzt sein können,
- R⁵: Cycloalkyl oder Cycloalkylalkylen mit 5-12 C-Atomen,
- R⁶: Phenyl oder Phenylmethyl,
- R⁷: COOH, COOA, CONH₂, CONHA, CON(A)₂ oder CN,
- A: Alkyl mit 1 bis 6 C-Atomen und
- Hal: F, Cl, Br oder I
bedeuten,
sowie deren physiologisch unbedenklichen Salze und/oder Solvate,
zur Herstellung eines Arzneimittels zur Behandlung von erektiler Dysfunktion bei Männern, die gleichzeitig mit Vasodilatatoren, die über das NO-cGMP-System wirken, behandelt werden.

Die Verwendung von PDE V-Hemmem ist beschrieben z.B. in der WO 94/28902.
Pyrimidinderivate sind beispielsweise aus der EP 201 188 oder der WO 93/06104 bekannt.
Die Verwendung von PDE (Phosphodiesterase) V Inhibitoren zur Behandlung erektiler Dysfunktion ist z.B. bekannt aus der EP 702 555 oder der WO 99/55708.

Bekanntester Vertreter der PDE V Inhibitoren ist Sildenafil (Viagra®).
Als Nebenwirkungen bei der Behandlung erektiler Dysfunktion durch PDE V Inhibitoren können cardiovaskuläre Nebenwirkungen wie Blutdrucksenkung und reflektorische Herzfrequenzerhöhung, insbesondere bei Nitratinteraktionen beobachtet werden.
So ist bei Nitratgabe die Anwendung von Sildenafil kontraindiziert.

Die Nitratgabe verstärkt hämodynamische Nebenwirkungen von Penis - unspezifischen PDE V - Inhibitoren.
Bekannte Nitratverbindungen sind z.B. Nitroglycerin = Nitrolingual® oder Isoket®.

Der Erfindung lag die Aufgabe zugrunde, neue Verbindungen mit wertvollen Eigenschaften aufzufinden, insbesondere solche, die zur Herstellung von Arzneimitteln verwendet werden können.

Überraschenderweise wurde gefunden, daß Penis-hochspezifische PDE V - Inhibitoren, oder deren physiologisch unbedenklichen Salze und/oder Solvate, zur Behandlung von erektiler Dysfunktion bei Männern, verwendet werden können, ohne daß dabei durch PDE V Inhibitoren hervorgerufene Kreislaufnebenwirkungen auftreten.
Insbesondere treten dabei keine Kreislaufnebenwirkungen von Penisunspezifischen PDE V Inhibitoren auf.

Diese Nebenwirkungen können insbesondere dann auftreten, wenn gleichzeitig Vasodilatatoren, die über das NO-cGMP-System wirken, verabreicht werden.

Bevorzugt sind dabei Vasodilatatoren ausgewählt aus der Gruppe der Nitratverbindungen.

Gegenstand der Erfindung ist insbesondere die Verwendung wie beschrieben, wobei der PDE V - Inhibitor ausgewählt ist aus der Gruppe
(a) 3-[4-(3-Chlor-4-methoxy-benzylamino)-benzo[4,5]thieno-[2,3-d]-pyrimidin-2-yl]-propionsäure;
(b) 4-[4-(3,4-Methylendioxy-benzylamino)-benzo[4,5]thieno-[2,3-d]-pyrimidin-2-yl]-buttersäure;
(c) 7-[4-(3,4-Methylendioxy-benzylamino)-benzo[4,5]thieno-[2,3-d]-pyrimidin-2-yl]-heptansäure;
(d) 7-[4-(3-Chlor-4-methoxy-benzylamino)-benzo[4,5]thieno-[2,3-d]-pyrimidin-2-yl]-heptansäure;
(e) 5-[4-(3-Chlor-4-methoxy-benzylamino)-benzo[4,5]thieno-[2,3-d]-pyrimidin-2-yl]-valeriansäure;
(f) 2-{4-[4-(3-Chlor-4-methoxy-benzylamino)-benzo[4,5]thieno-[2,3-d]-pyrimidin-2-yl]-cyclohexyl-1-yl}-essigsäure;
(g) 4-[4-(3,4-Methylendioxy-benzylamino)-benzo[4,5]thieno-[2,3-d]-pyrimidin-2-yl]-cyclohexancarbonsäure;
(h) 4-[4-(3,4-Methylendioxy-benzylamino)-benzo[4,5]thieno-[2,3-d]-pyrimidin-2-yl]-benzoesäure;
(i) 4-[4-(3,4-Methylendioxy-benzylamino)-benzo[4,5]thieno-[2,3-d]-pyrimidin-2-yl]-phenylessigsäure;
(k) 4-[4-(3-Chlor-4-methoxy-benzylamino)-benzo[4,5]thieno-[2,3-d]-pyrimidin-2-yl]-cyclohexancarbonsäure, Ethanolaminsalz;
sowie deren physiologisch unbedenklichen Salze und/oder Solvate.

Bei den beschriebenen Verbindungen der Formel I erfolgt die selektive Wirkung spezifisch am Penis
a) durch Inhibierung eines Penis-spezifischen Subtyps der Phosphodiesterase V, und/oder
b) als Folge eines selektiven Transports in die Peniseffektorzelle bzw. durch schnelle Elimination aus den Effektorzellen des Herzkreislaufsystems.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Vor- und nachstehend haben die Reste R¹, R², R³, R⁴, R⁵, R⁶, R⁷, X und L die bei den Formeln I, II und III angegebenen Bedeutungen, sofern nicht ausdrücklich etwas anderes angegeben ist.

A bedeutet Alkyl mit 1-6 C-Atomen.
In den vorstehenden Formeln ist Alkyl vorzugsweise unverzweigt und hat 1, 2, 3, 4, 5 oder 6 C-Atome und bedeutet vorzugsweise Methyl, Ethyl oder Propyl, weiterhin bevorzugt Isopropyl, Butyl, Isobutyl, sek.-Butyl oder tert.-Butyl, aber auch n-Pentyl, Neopentyl, Isopentyl oder Hexyl.

X bedeutet einen einfach durch R⁷ substituierten R⁴-, R⁵- oder R⁶-Rest.

R⁴ bedeutet einen linearen oder verzweigten Alkylenrest mit 1-10 C-Atomen, wobei der Alkylenrest vorzugsweise z.B. Methylen, Ethylen, Propylen, Isopropylen, Butylen, Isobutylen, sek.-Butylen, Pentylen, 1-, 2- oder 3-Methylbutylen, 1,1- , 1,2- oder 2,2-Dimethylpropylen, 1-Ethylpropylen, Hexylen, 1- , 2- , 3- oder 4-Methylpentylen, 1,1- , 1,2- , 1,3- , 2,2- , 2,3- oder 3,3-Dimethylbutylen, 1- oder 2-Ethylbutylen, 1-Ethyl-1-methylpropylen, 1-Ethyl-2-methylpropylen, 1,1,2- oder 1,2,2-Trimethylpropylen, lineares oder verzweigtes Heptylen, Octylen, Nonylen oder Decylen bedeutet.
R⁵ bedeutet ferner z.B. But-2-en-ylen oder Hex-3-en-ylen.
Ganz besonders bevorzugt ist Ethylen, Propylen oder Butylen.

R⁵ bedeutet Cycloalkylalkylen mit 5-12 C-Atomen, vorzugsweise z.B. Cycclopentylmethylen, Cyclohexylmethylen, Cyclohexylethylen, Cyclohexylpropylen oder Cyclohexylbutylen.
R⁵ bedeutet auch Cycloalkyl mit vorzugsweise mit 5-7 C-Atomen. Cycloalkyl bedeutet z.B. Cyclopentyl, Cyclohexyl oder Cycloheptyl.

Hal bedeutet vorzugsweise F, Cl oder Br, aber auch I.

Die Reste R¹ und R² können gleich oder verschieden sein und stehen vorzugsweise in der 3- oder 4-Position des Phenylrings. Sie bedeuten beispielsweise jeweils unabhängig voneinander H, Alkyl, F, Cl, Br oder I oder zusammen Alkylen, wie z.B. Propylen, Butylen oder Pentylen, ferner Ethylenoxy, Methylendioxy oder Ethylendioxy. Bevorzugt stehen sie auch jeweils für Alkoxy, wie z.B. für Methoxy, Ethoxy oder Propoxy, ferner für Hydroxy.

Der Rest R⁷ bedeutet vorzugsweise z.B. COOH, COOCH₃, COOC₂H₅, CONH₂, CON(CH₃)₂, CONHCH₃ oder CN.

Für die gesamte Erfindung gilt, daß sämtliche Reste, die mehrfach auftreten, gleich oder verschieden sein können, d.h. unabhängig voneinander sind.

Dementsprechend ist Gegenstand der Erfindung insbesondere die Verwendung derjenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Id ausgedrückt werden, die der Formel I entsprechen und worin die nicht näher bezeichneten Reste die bei der Formel I angegebene Bedeutung haben, worin jedoch
- in Ia: X durch COOH, COOA, CONH₂, CONA₂, CONHA oder CN substituiertes R⁴, Phenyl oder Phenylmethyl bedeuten;
- in Ib: R¹ und R² zusammen Alkylen mit 3-5 C-Atomen, -O-CH₂-CH₂-, -O-CH₂-O- oder -O-CH₂-CH₂-O,
X durch COOH, COOA, CONH₂, CONA₂, CONHA oder CN substituiertes R⁴, Phenyl oder Phenylmethyl bedeuten;
- in Ic: R¹, R² jeweils unabhängig voneinander H, A, OA oder Hal,
R¹ und R² zusammen Alkylen mit 3-5 C-Atomen, -O-CH₂-CH₂-, -O-CH₂-O- oder -O-CH₂-CH₂-O,
X durch COOH, COOA, CONH₂, CONA₂, CONHA oder CN substituiertes R⁴, Phenyl oder Phenylmethyl bedeuten;
- in Id: R¹, R² jeweils unabhängig voneinander H, A, OA oder Hal,
R¹ und R² zusammen auch Alkylen mit 3-5 C-Atomen, -O-CH₂-CH₂-, -O-CH₂-O- oder -O-CH₂-CH₂-O-,
X einfach durch R⁷ substituiertes Alkylen mit 2-5 C-- Atomen, Cyclohexyl, Phenyl oder Phenylmethyl,
R⁷ COOH oder COOA,
A Alkyl mit 1 bis 6 C-Atomen,
Hal F, Cl, Br oder I bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II oder III haben R¹, R², R³, R⁴, X und n die angegebenen Bedeutungen, insbesondere die angegebenen bevorzugten Bedeutungen.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy, ferner auch 2-Naphthalinsulfonyloxy).

Die Verbindungen der Formel I können vorzugsweise erhalten werden, indem man Verbindungen der Formel II mit Verbindungen der Formel III umsetzt.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Ausgangsverbindungen der Formel II und III sind in der Regel bekannt. Sind sie nicht bekannt, so können sie nach an sich bekannten Methoden hergestellt werden.
Verbindungen der Formel II können z.B. durch Umsetzung mit POCl₃ aus den entsprechenden Hydroxypyrimidinen erhalten werden, die aus Thiophenderivaten und CN-substituierten Alkylencarbonsäureestern aufgebaut werden (Eur. J. Med. Chem. 23, 453 (1988)).
Die Darstellung der Hydroxypyrimidine erfolgt entweder durch Dehydrierung entsprechender Tetrahydrobenzthienopyrimidinverbindungen oder nach der für die Herstellung von Pyrimidinderivaten üblichen Cyclisierung von 2-Aminobenzthiophen-3-carbonsäure-derivaten mit Aldehyden oder Nitrilen (z.B. Houben Weyl E9b/2).

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Es ist ferner möglich, in einer Verbindung der Formel I einen Rest X in einen anderen Rest X umzuwandeln, z.B. indem man einen Ester oder eine Cyangruppe zu einer COOH-Gruppe hydrolysiert.
Estergruppen können z.B. mit NaOH oder KOH in Wasser, Wasser-THF oder Wasser-Dioxan bei Temperaturen zwischen 0 und 100° verseift werden.
Carbonsäuren können z.B. mit Thionylchlorid in die entsprechenden Carbonsäurechloride und diese in Carbonsäureamide umgewandelt werden. Durch Wasserabspaltung in bekannter Weise erhält man aus diesen Carbonitrile.

Eine Säure der Formel I kann mit einer Base in das zugehörige Säureadditionssalz übergeführt werden, beispielsweise durch Umsetzung äquivalenter Mengen der Säure und der Base in einem inerten Lösungsmittel wie Ethanol und anschließendes Eindampfen. Für diese Umsetzung kommen insbesondere Basen in Frage, die physiologisch unbedenkliche Salze liefern.
So kann die Säure der Formel I mit einer Base (z.B. Natrium- oder Kaliumhydroxid oder -carbonat) in das entsprechende Metall-, insbesondere Alkalimetall- oder Erdalkalimetall-, oder in das entsprechende Ammoniumsalz umgewandelt werden.
Für diese Umsetzung kommen insbesondere auch organische Basen in Frage, die physiologisch unbedenkliche Salze liefern, wie z.B. Ethanolamin.

Andererseits kann eine Base der Formel I mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure. Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

### Pharmakologischer Testbericht

Untersuchungen von trans-4-[4-(3-Chlor-4-methoxy-benzylamino)-benzo[4,5]thieno-[2,3-d]-pyrimidin-2-yl]-cyclohexancarbonsäure, Ethanolaminsalz (EMD 221829) zur Hämodynamik und zur erektilen Funktion in verschiedenen Tierspezies zum Teil unter gleichzeitiger Nitratgabe im Vergleich zu Sildenafil Citrat.

Alle Dosisangaben i.d. (intraduodenal), falls nicht anders angegeben.

### A

### Messungen zur erektilen Funktion an anästhesierten Hunden mit geschlossenem Thorax

Erektionen unterschiedlichen Ausmaßes wurden durch Stimulationsvariation des Nervus Pelvinus ausgelöst. Gemessen wurde der Druck im corpus cavernosum rigidum [CCR].

### Ergebnis:

EMD 221829 potenziert submaximale Erektion noch bei 1 mg Substanz / 1 kg Körpergewicht.
Für Sildenafil werden entsprechende Ergebnisse gemessen.

Gleichzeitig wurden Blutdruck und Herzfrequenz gemessen.
Im Gegensatz zu Sildenafil ist bei EMD 221829 bei 1 mg/kg keine Beeinflussung dieser hämodynamischen Parameter feststellbar.

### B

Extensive Untersuchungen zur Hämodynamik von EMD 221829 an anästhesierten Hunden mit offenem Thorax zeigten keine cardiovaskulären Nebenwirkungen, wie z.B. Blutdrucksenkung mit reflektorischem Herzfrequenzanstieg.
Bei 1 mg Substanz / 1 kg Körpergewicht ist ein deutlicher Unterschied der hämodynamischen Parameter im Vergleich zu Sildenafil zu messen.

Bei EMD 221829 werden erst bei 100 mg Substanz / 1 kg Körpergewicht Effekte auf Blutdruck und Herzfrequenz messbar. In dieser Dosis führt Sildenafil Citrat zu einer Blutdrucksenkung und Herzfrequenzerhöhung.

### C

### Untersuchungen zur Nitratinteraktion von EMD 221829 bzw. Sildenafil

Modell und Applikation wie bei **B,** allerdings erfolgt die Gabe beider Testsubstanzen bei gleichzeitiger permanenter i.v. - Infusion von Isosorbit Dinitrat (50 µg / kg Körpergewicht / Stunde).

### Ergebnis:

Die Sildenafil-induzierten Veränderungen der o.g. Parameter werden durch gleichzeitige Nitratgabe signifikant potenziert. Im Gegensatz dazu zeigen sich bei EMD 221829 unter den gleichen Bedingungen keine Veränderungen der Ausgangsparameter.

### D

### Telemetrische Messungen von Blutdruck und Herzfrequenz über 24 Stunden in spontan hypertensiven Ratten

Messungen 3, 10, 30 mg Substanz / 1 kg Körpergewicht / p.o.

Nach Sildenafil-Gabe ergibt sich eine dosisabhängige signifikante Reduktion des Blutdrucks. Bei EMD 221829 ist im gleichen Dosisbereich keine Veränderung des Blutdrucks nachweisbar.

### E

### wie D an normotensiven Wistar-Ratten

Messungen 30, 100, 300 mg Substanz / 1 kg Körpergewicht / p.o.

### Ergebnis:

Nach Sildenafil-Gabe ergibt sich eine signifikante dosisabhängige Reduktion des Blutdrucks und eine Steigerung der Herzfrequenz.
Bei Gabe von EMD 221829 sind keine Veränderungen feststellbar.

### F

### Nitratinteraktion in spontan hypertensiven Ratten

Methode: chirurgische Instrumentierung und Messung von Blutdruck und Herzfrequenz nach intravenöser kummulativer Applikation von entweder EMD 221829 oder Sildenafil (0.1, 0.3, 1, 3, 10 mg / kg Körpergewicht) bei gleichzeitiger Infusion von Isosorbit-Dinitrat (100 µg / kg Körpergewicht / Stunde).

Ergebnis: Beobachtet wird ein dosisabhängiger Blutdruckabfall und Herzfrequenz-Anstieg bei Sildenafil, wohingegen bei EMD 221829 keine Veränderung beobachtet wird.

### Fazit:

Die pharmakologischen Untersuchungen belegen die Wirksamkeit von Penis-hochspezifischen PDE V - Inhibitoren, die eine Behandlung der erektilen Dysfunktion bei Männern und sexueller Störungen bei Frauen ohne negative Beeinflussung des Herz-Kreislauf-systems, insbesondere bei gleichzeitiger Nitratbehandlung, ermöglichen.

### Herstellung der Verbindungen

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

| | |
|---|---|
| Massenspektrometrie (MS) | EI (Elektronenstoß-Ionisation) M⁺ |
| | FAB (Fast Atom Bombardment) (M+H)⁺ |

### Beispiel 1

3-(4-Chlor-benzothieno-[2,3-d]-pyrimidin-2-yl)-propionsäuremethylester [erhältlich durch Cyclisierung von 2-Amino-5,6,7,8-tetrahydrobenzothiophen-3-carbonsäuremethylester mit 3-Cyanpropionsäuremethylester, Dehydrierung mit Schwefel und nachfolgender Chlorierung mit Phosphoroxichlorid/Dimethylamin] und 3-Chlor-4-methoxybenzylamin ("A") in N-Methylpyrrolidon werden 5 Stunden bei 110° gerührt. Das Lösungsmittel wird entfernt und wie üblich aufgearbeitet. Man erhält 3-[4-(3-Chlor-4-methoxy-benzylamino)-benzothieno-[2,3-d]-pyrimidin-2-yl]-propionsäuremethylester als farbloses Öl.
Analog erhält man durch Umsetzung von "A"
   mit 2-(4-Chlor-benzothieno-[2,3-d]-pyrimidin-2-yl)-essigsäuremethylester
      2-[4-(3-Chlor-4-methoxy-benzylamino)-benzothieno-[2,3-d]-pyrimidin-2-yl]-essigsäuremethylester.
Analog erhält man durch Umsetzung von 3,4-Methylendioxybenzylamin
   mit 3-(4-Chlor-benzothieno-[2,3-d]-pyrimidin-2-yl)-propionsäuremethylester
      3-[4-(3,4-Methylendioxy-benzylamino)-benzothieno-[2,3-d]-pyrimidin-2-yl]-propionsäuremethylester.
Analog erhält man durch Umsetzung von "A"
   mit 4-(4-Chlor-benzothieno-[2,3-d]-pyrimidin-2-yl)-buttersäuremethylester
      4-[4-(3-Chlor-4-methoxy-benzylamino)-benzothieno-[2,3-d]-pyrimidin-2-yl]-buttersäuremethylester.
Analog erhält man durch Umsetzung von 3,4-Methylendioxybenzylamin
   mit 4-(4-Chlor-benzothieno-[2,3-d]-pyrimidin-2-yl)-buttersäuremethylester
      4-[4-(3,4-Methylendioxy-benzylamino)-benzothieno-[2,3-d]-pyrimidin-2-yl]-buttersäuremethylester.
Analog erhält man durch Umsetzung von "A"
   mit 5-(4-Chlor-benzothieno-[2,3-d]-pyrimidin-2-yl)-valeriansäuremethylester
      5-[4-(3-Chlor-4-methoxy-benzylamino)-benzothieno-[2,3-d]-pyrimidin-2-yl]-valeriansäuremethylester.
Analog erhält man durch Umsetzung von 3,4-Methylendioxybenzylamin
   mit 5-(4-Chlor-benzothieno-[2,3-d]-pyrimidin-2-yl)-valeriansäuremethylester
      5-[4-(3,4-Methylendioxy-benzylamino)-benzothieno-[2,3-d]-pyrimidin-2-yl]-valeriansäuremethylester.
Analog erhält man durch Umsetzung von "A"
   mit 7-(4-Chlor-benzothieno-[2,3-d]-pyrimidin-2-yl)-heptansäuremethylester
      7-[4-(3-Chlor-4-methoxy-benzylamino)-benzothieno-[2,3-d]-pyrimidin-2-yl]-heptansäuremethylester.
Analog erhält man durch Umsetzung von 3,4-Methylendioxybenzylamin
   mit 7-(4-Chlor-benzothieno-[2,3-d]-pyrimidin-2-yl)-heptansäuremethylester
      7-[4-(3,4-Methylendioxy-benzylamino)-benzothieno-[2,3-d]-pyrimidin-2-yl]-heptansäuremethylester.
Analog erhält man durch Umsetzung von "A"
   mit 2-[4-(4-Chlor-benzothieno-[2,3-d]-pyrimidin-2-yl)-cyclohex-1-yl]-essigsäuremethylester
      2-{4-[4-(3-Chlor-4-methoxy-benzylamino)-benzothieno-[2,3-d]-pyrimidin-2-yl]-cyclohexyl-1-yl}-essigsäuremethylester.
Analog erhält man durch Umsetzung von 3,4-Methylendioxybenzylamin
   mit 2-[4-(4-Chlor-benzothieno-[2,3-d]-pyrimidin-2-yl)-cyclohex-1-yl]-essigsäuremethylester
      2-{4-[4-(3,4-Methylendioxy-benzylamino)-benzothieno-[2,3-d]-pyrimidin-2-yl]-cyclohexyl-1-yl}-essigsäuremethylester.
Analog erhält man durch Umsetzung von Benzylamin
   mit 3-(4-Chlor-benzothieno-[2,3-d]-pyrimidin-2-yl)-propionsäuremethylester
      3-(4-Benzylamino-benzothieno-[2,3-d]-pyrimidin-2-yl)-propionsäuremethylester;
   mit 4-(4-Chlor-benzothieno-[2,3-d]-pyrimidin-2-yl)-buttersäuremethylester
      4-(4-Benzylamino-benzothieno-[2,3-d]-pyrimidin-2-yl)-buttersäuremethylester;
   mit 5-(4-Chlor-benzothieno-[2,3-d]-pyrimidin-2-yl)-valeriansäuremethylester
      5-(4-Benzylamino-benzothieno-[2,3-d]-pyrimidin-2-yl)-valeriansäuremethylester.
Analog erhält man durch Umsetzung von "A"
   mit 4-(4-Chlor-benzothieno-[2,3-d]-pyrimidin-2-yl)-cyclohexancarbonsäuremethylester
      4-[4-(3-Chlor-4-methoxy-benzylamino)-benzothieno-[2,3-d]-pyrimidin-2-yl]-cyclohexancarbonsäuremethylester
   und durch Umsetzung von 3,4-Methylendioxybenzylamin
      4-[4-(3,4-Methylendioxy-benzylamino)-benzothieno-[2,3-d]-pyrimidin-2-yl]-cyclohexancarbonsäuremethylester.

### Beispiel 2

3-[4-(3-Chlor-4-methoxy-benzylamino)-benzothieno-[2,3-d]-pyrimidin-2-yl]-propionsäuremethylester wird in Ethylenglycolmonomethylether gelöst und nach Zugabe von 32 %iger NaOH 5 Stunden bei 110° gerührt. Nach Zugabe von 20 %iger HCl wird mit Dichlormethan extrahiert. Durch Zugabe von Petrolether erhält man 3-[4-(3-Chlor-4-methoxy-benzylamino)-benzothieno-[2,3-d]-pyrimidin-2-yl]-propionsäure, F. 218°.

Die ausgefallenen Kristalle werden in Isopropanol gelöst und mit Ethanolamin versetzt. Nach Kristallisation erhält man 3-[4-(3-Chlor-4-methoxy-benzylamino)-benzothieno-[2,3-d]-pyrimidin-2-yl]-propionsäure, Ethanolaminsalz.
Analog erhält man die Verbindungen
   4-[4-(3-Chlor-4-methoxy-benzylamino)-benzothieno-[2,3-d]-pyrimidin-2-yl]-buttersäure, F. 225°; Ethanolaminsalz F. 150°;
   5-[4-(3-Chlor-4-methoxy-benzylamino)-benzothieno-[2,3-d]-pyrimidin-2-yl]-valeriansäure, F. 210°; Ethanolaminsalz F. 141°;
   4-[4-(3,4-Methylendioxy-benzylamino)-benzothieno-[2,3-d]-pyrimidin-2-yl]-buttersäure, Hydrochlorid, F. 245°.
Analog erhält man aus den unter Beispiel 1 aufgeführten Estern die nachstehenden Carbonsäuren:
   2-[4-(3-Chlor-4-methoxy-benzylamino)-benzothieno-[2,3-d]-pyrimidin-2-yl]-essigsäure,
   3-[4-(3,4-Methylendioxy-benzylamino)-benzothieno-[2,3-d]-pyrimidin-2-yl]-propionsäure,
   5-[4-(3,4-Methylendioxy-benzylamino)-benzothieno-[2,3-d]-pyrimidin-2-yl]-valeriansäure,
   7-[4-(3-Chlor-4-methoxy-benzylamino)-benzothieno-[2,3-d]-pyrimidin-2-yl]-heptansäure,
   7-[4-(3,4-Methylendioxy-benzylamino)-benzothieno-[2,3-d]-pyrimidin-2-yl]-heptansäure,
   2-{4-[4-(3-Chlor-4-methoxy-benzylamino)-benzothieno-[2,3-d]-pyrimidin-2-yl]-cyclohexyl-1-yl}-essigsäure,
   2-{4-[4-(3,4-Methylendioxy-benzylamino)-benzothieno-[2,3-d]-pyrimidin-2-yl]-cyclohexyl-1-yl}-essigsäure,
   3-(4-Benzylamino-benzothieno-[2,3-d]-pyrimidin-2-yl)-propionsäure,
   4-(4-Benzylamino-benzothieno-[2,3-d]-pyrimidin-2-yl)-buttersäure,
   5-(4-Benzylamino-benzothieno-[2,3-d]-pyrimidin-2-yl)-valeriansäure,
   4-[4-(3-Chlor-4-methoxy-benzylamino)-benzothieno-[2,3-d]-pyrimidin-2-yl]-cyclohexancarbonsäure, Ethanolaminsalz, F. 167°;
   4-[4-(3,4-Methylendioxy-benzylamino)-benzothieno-[2,3-d]-pyrimidin-2-yl]-cyclohexancarbonsäure, Ethanolaminsalz, F. 143°.

### Beispiel 3

Eine Mischung von 1,5 g 4-(4-Chlorbenzothieno-[2,3-d]-pyrimidin-2-yl)-phenylcarbonsäuremethylester ("B"), hergestellt durch Dehydrierung der entsprechenden 5,6,7,8-Tetrahydrobenzthieno-[2,3-d]-pyrimidinverbindung mit Schwefel und nachfolgender Chlorierung mit Phosphoroxichlorid / Dimethylamin, und 1,5 g 3-Chlor-4-methoxy-benzylamin in 20 ml N-Methylpyrrolidon wird 4 Stunden auf 110° erwärmt. Nach dem Abkühlen wird wie ünlich aufgearbeitet. Man erhält 2,6 g 4-[4-(3-Chlor-4-methoxy-benzylamino)-[1]benzothieno-[2,3-d]-pyrimidin-2-yl]-benzoesäuremethylester, F. 203-204°.
Analog Beispiel 2 erhält man aus 1,2 g des Esters daraus 1,0 g
   4-[4-(3-Chlor-4-methoxy-benzylamino)-[1]benzothieno-[2,3-d]-pyrimidin-2-yl]-benzoesäure, Ethanolaminsalz F. 189-190°.
Analog Beispiel 1 erhält man aus "B" und 3,4-Methylendioxybenzylamin
   4-[4-(3,4-Methylendioxy-benzylamino)-[1]benzothieno-[2,3-d]-pyrimidin-2-yl]-benzoesäuremethylester und daraus durch Esterhydrolyse
   4-[4-(3,4-Methylendioxy-benzylamino)-[1]benzothieno-[2,3-d]-pyrimidin-2-yl]-benzoesäure, Natriumsalz, F. >260°.
Analog erhält man die Verbindung
   4-[4-(3-Chlor-4-methoxy-benzylamino)-[1]benzothieno-[2,3-d]-pyrimidin-2-yl]-phenylessigsäure, Ethanolaminsalz, F. 130°; und
   4-[4-(3,4-Methylendioxy-benzylamino)-[1]benzothieno-[2,3-d]-pyrimidin-2-yl]-phenylessigsäure, Ethanolaminsalz, F. 202°.

### Beispiel 4

1 Äquivalent 3-[4-(3-Chlor-4-methoxy-benzylamino)-benzothieno-[2,3-d]-pyrimidin-2-yl]-propionsäure und 1,2 Äquivalente Thionylchlorid werden 2 Stunden in Dichlormethan gerührt. Das Lösungsmittel wird entfernt und man erhält 3-[4-(3-Chlor-4-methoxy-benzylamino)-benzothieno-[2,3-d]-pyrimidin-2-yl]-propionsäurechlorid.
Man überführt in wässriges Ammoniak, rührt eine Stunde und erhält nach üblicher Aufarbeitung 3-[4-(3-Chlor-4-methoxy-benzylamino)-benzothieno-[2,3-d]-pyrimidin-2-yl]-propionsäureamid.

### Beispiel 5

1 Äquivalent DMF und 1 Äquivalent Oxalylchlorid werden bei 0° in Acetonitril gelöst. Danach wird 1 Äquivalent 3-[4-(3-Chlor-4-methoxybenzylamino)-benzothieno-[2,3-d]-pyrimidin-2-yl]-propionsäureamid zugegeben. Es wird eine Stunde nachgerührt. Nach üblicher Aufarbeitung erhält man 3-[4-(3-Chlor-4-methoxy-benzylamino)-benzothieno-[2,3-d]-pyrimidin-2-yl]-propionitril.

### Beispiel 6

Analog den Beispielen 1, 2 und 3 erhält man durch Umsetzung der entsprechenden Chlor-pyrimidinderivate mit 3,4-Ethylendioxybenzylamin die nachstehenden Carbonsäuren
4-[4-(3,4-Ethylendioxy-benzylamino)-benzothieno-[2,3-d]-pyrimidin-2-yl]-buttersäure,
3-[4-(3,4-Ethylendioxy-benzylamino)-benzothieno-[2,3-d]-pyrimidin-2-yl]-propionsäure,
5-[4-(3,4-Ethylendioxy-benzylamino)-benzothieno-[2,3-d]-pyrimidin-2-yl]-valeriansäure,
7-[4-(3,4-Ethylendioxy-benzylamino)-benzothieno-[2,3-d]-pyrimidin-2-yl]-heptansäure,
2-{4-[4-(3,4-Ethylendioxy-benzylamino)-benzothieno-[2,3-d]-pyrimidin-2-yl]-cyclohexyl-1-yl}-essigsäure,
4-[4-(3,4-Ethylendioxy-benzylamino)-benzothieno-[2,3-d]-pyrimidin-2-yl]-cyclohexancarbonsäure,
4-[4-(3,4-Ethylendioxy-benzylamino)-[1]benzothieno-[2,3-d]-pyrimidin-2-yl]-benzoesäure, Zers. 220-230°;
4-[4-(3,4-Ethylendioxy-benzylamino)-[1]benzothieno-[2,3-d]-pyrimidin-2-yl]-benzoesäure, Ethanolaminsalz, F. 252°;
4-[4-(3,4-Ethylendioxy-benzylamino)-[1]benzothieno-[2,3-d]-pyrimidin-2-yl]-phenylessigsäure.

Analog erhält man durch Umsetzung mit 3,4-Dichlorbenzylamin die nachstehenden Verbindungen
4-[4-(3,4-Dichlor-benzylamino)-benzothieno-[2,3-d]-pyrimidin-2-yl]-buttersäure,
3-[4-(3,4-Dichlor-benzylamino)-benzothieno-[2,3-d]-pyrimidin-2-yl]-propionsäure,
5-[4-(3,4-Dichlor-benzylamino)-benzothieno-[2,3-d]-pyrimidin-2-yl]-valeriansäure, Ethanolaminsalz, F. 160°;
7-[4-(3,4-Dichlor-benzylamino)-benzothieno-[2,3-d]-pyrimidin-2-yl]-heptansäure,
2-{4-[4-(3,4-Dichlor-benzylamino)-benzothieno-[2,3-d]-pyrimidin-2-yl]-cyclohexyl-1-yl}-essigsäure,
4-[4-(3,4-Dichlor-benzylamino)-benzothieno-[2,3-d]-pyrimidin-2-yl]-cyclohexancarbonsäure,
4-[4-(3,4-Dichlor-benzylamino)-[1]benzothieno-[2,3-d]-pyrimidin-2-yl]-benzoesäure,
4-[4-(3,4-Dichlor-benzylamino)-[1]benzothieno-[2,3-d]-pyrimidin-2-yl]-phenylessigsäure.

Analog erhält man durch Umsetzung mit 3-Chlor-4-ethoxybenzylamin die nachstehenden Verbindungen
4-[4-(3-Chlor-4-ethoxybenzylamino)-benzothieno-[2,3-d]-pyrimidin-2-yl]-buttersäure,
3-[4-(3-Chlor-4-ethoxybenzylamino)-benzothieno-[2,3-d]-pyrimidin-2-yl]-propionsäure,
5-[4-(3-Chlor-4-ethoxybenzylamino)-benzothieno-[2,3-d]-pyrimidin-2-yl]-valeriansäure,
7-[4-(3-Chlor-4-ethoxybenzylamino)-benzothieno-[2,3-d]-pyrimidin-2-yl]-heptansäure,
2-{4-[4-(3-Chlor-4-ethoxybenzylamino)-benzothieno-[2,3-d]-pyrimidin-2-yl]-cyclohexyl-1-yl}-essigsäure,
4-[4-(3-Chlor-4-ethoxybenzylamino)-benzothieno-[2,3-d]-pyrimidin-2-yl]-cyclohexancarbonsäure,
4-[4-(3-Chlor-4-ethoxybenzylamino)-[1]benzothieno-[2,3-d]-pyrimidin-2-yl]-benzoesäure, F. 185-187°;
4-[4-(3-Chlor-4-ethoxybenzylamino)-[1]benzothieno-[2,3-d]-pyrimidin-2-yl]-phenylessigsäure.

Analog erhält man durch Umsetzung mit 3-Chlor-4-isopropoxybenzylamin die nachstehenden Verbindungen
4-[4-(3-Chlor-4-isopropoxybenzylamino)-benzothieno-[2,3-d]-pyrimidin-2-yl]-buttersäure,
3-[4-(3-Chlor-4-isopropoxybenzylamino)-benzothieno-[2,3-d]-pyrimidin-2-yl]-propionsäure,
5-[4-(3-Chlor-4-isopropoxybenzylamino)-benzothieno-[2,3-d]-pyrimidin-2-yl]-valeriansäure, Ethanolaminsalz, F. 130°;
7-[4-(3-Chlor-4-isopropoxybenzylamino)-benzothieno-[2,3-d]-pyrimidin-2-yl]-heptansäure,
2-{4-[4-(3-Chlor-4-isopropoxybenzylamino)-benzothieno-[2,3-d]-pyrimidin-2-yl]-cyclohexyl-1-yl}-essigsäure,
4-[4-(3-Chlor-4-isopropoxybenzylamino)-benzothieno-[2,3-d]-pyrimidin-2-yl]-cyclohexancarbonsäure,
4-[4-(3-Chlor-4-isopropoxybenzylamino)-[1]benzothieno-[2,3-d]-pyrimidin-2-yl]-benzoesäure, F. 240-241°;
4-[4-(3-Chlor-4-isopropoxybenzylamino)-[1]benzothieno-[2,3-d]-pyrimidin-2-yl]-phenylessigsäure.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verwendung von PDE V - Inhibitoren der Formel I 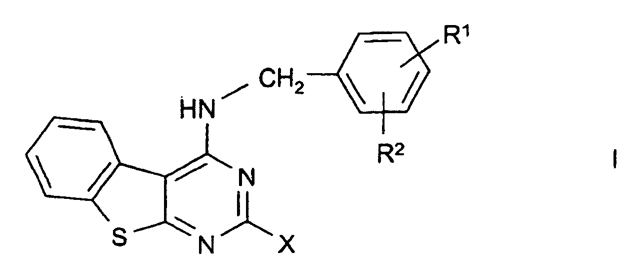 worin
R¹, R² jeweils unabhängig voneinander H, A, OA, OH oder Hal,
R¹ und R² zusammen auch Alkylen mit 3-5 C-Atomen, -O-CH₂-CH₂-, -CH₂-O-CH₂-, -O-CH₂-O- oder -O-CH₂-CH₂-O-,
X einfach durch R⁷ substituiertes R⁴, R⁵ oder R⁶,
R⁴ lineares oder verzweigtes Alkylen mit 1-10 C-Atomen, worin eine oder zwei CH₂-Gruppen durch -CH=CH-Gruppen ersetzt sein können,
R⁵ Cycloalkyl oder Cycloalkylalkylen mit 5-12 C-Atomen,
R⁶ Phenyl oder Phenylmethyl,
R⁷ COOH, COOA, CONH₂, CONHA, CON(A)₂ oder CN,
A Alkyl mit 1 bis 6 C-Atomen und
Hal F, Cl, Br oder I
bedeuten,
sowie deren physiologisch unbedenklichen Salze und/oder Solvate, zur Herstellung eines Arzneimittels zur Behandlung von erektiler Dysfunktion bei Männern, die gleichzeitig mit Vasodilatatoren, die über das NO-cGMP-System wirken, behandelt werden.

2. Verwendung nach Anspruch 1, wobei die Vasodilatatoren aus der Gruppe der Nitratverbindungen ausgewählt sind.

3. Verwendung nach einem der vorhergehenden Ansprüche, wobei der PDE V - Inhibitor ausgewählt ist aus der Gruppe
(a) 3-[4-(3-Chlor-4-methoxy-benzylamino)-benzo[4,5]thieno-[2,3-d]-pyrimidin-2-yl]-propionsäure;
(b) 4-[4-(3,4-Methylendioxy-benzylamino)-benzo[4,5]thieno-[2,3-d]-pyrimidin-2-yl]-buttersäure;
(c) 7-[4-(3,4-Methylendioxy-benzylamino)-benzo[4,5]thieno-[2,3-d]-pyrimidin-2-yl]-heptansäure;
(d) 7-[4-(3-Chlor-4-methoxy-benzylamino)-benzo[4,5]thieno-[2,3-d]-pyrimidin-2-yl]-heptansäure;
(e) 5-[4-(3-Chlor-4-methoxy-benzylamino)-benzo[4,5]thieno-[2,3-d]-pyrimidin-2-yl]-valeriansäure;
(f) 2-{4-[4-(3-Chlor-4-methoxy-benzylamino)-benzo[4,5]thieno-[2,3-d]-pyrimidin-2-yl]-cyclohexyl-1-yl}-essigsäure;
(g) 4-[4-(3,4-Methylendioxy-benzylamino)-benzo[4,5]thieno-[2,3-d]-pyrimidin-2-yl]-cyclohexancarbonsäure;
(h) 4-[4-(3,4-Methylendioxy-benzylamino)-benzo[4,5]thieno-[2,3-d]-pyrimidin-2-yl]-benzoesäure;
(i) 4-[4-(3,4-Methylendioxy-benzylamino)-benzo[4,5]thieno-[2,3-d]-pyrimidin-2-yl]-phenylessigsäure;
(k) 4-[4-(3-Chlor-4-methoxy-benzylamino)-benzo[4,5]thieno-[2,3-d]-pyrimidin-2-yl]-cyclohexancarbonsäure, Ethanolaminsalz;
sowie deren physiologisch unbedenklichen Salze und/oder Solvate.

## Claims

1. Use of PDE V inhibitors of the formula I 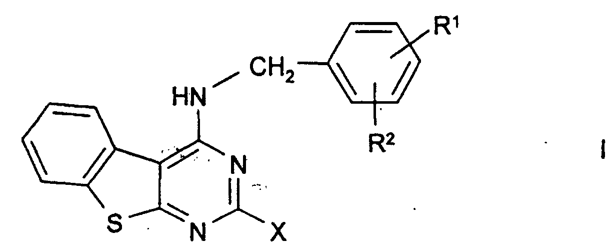 in which
R¹, R² each, independently of one another, denote H, A, OA, OH or Hal,
R¹ and R² together also denote alkylene having 3-5 C atoms, -O-CH₂-CH₂-, -CH₂-O-CH₂-, -O-CH₂-O- or -O-CH₂-CH₂-O-,
X denotes mono-R⁷-substituted R⁴, R⁵ or R⁶,
R⁴ denotes linear or branched alkylene having 1-10 C atoms, in which one or two CH₂ groups may be replaced by -CH=CH- groups,
R⁵ denotes cycloalkyl or cycloalkylalkylene having 5-12 C atoms,
R⁶ denotes phenyl or phenylmethyl,
R⁷ denotes COOH, COOA, CONH₂, CONHA, CON(A)₂ or CN,
A denotes alkyl having 1 to 6 C atoms and
Hal denotes F, Cl, Br or I,
and physiologically acceptable salts and/or solvates thereof, for the preparation of a medicament for the treatment of erectile dysfunction in men who are simultaneously being treated with vasodilators which act via the NO-cGMP system.

2. Use according to Claim 1, where the vasodilators are selected from the group of the nitrate compounds.

3. Use according to one of the preceding claims, where the PDE V inhibitor is selected from the group
(a) 3-[4-(3-chloro-4-methoxybenzylamino)benzo[4,5]thieno[2,3-d]-pyrimidin-2-yl]propionic acid;
(b) 4-[4-(3,4-methylenedioxybenzylamino)benzo[4,5]thieno[2,3-d]-pyrimidin-2-yl]butyric acid;
(c) 7-[4-(3,4-methylenedioxybenzylamino)benzo[4,5]thieno[2,3-d]-pyrimidin-2-yl]heptanoic acid;
(d) 7-[4-(3-chloro-4-methoxybenzylamino)benzo[4,5]thieno[2,3-d]-pyrimidin-2-yl]heptanoic acid;
(e) 5-[4-(3-chloro-4-methoxybenzylamino)benzo[4,5]thieno[2,3-d]-pyrimidin-2-yl]valeric acid;
(f) 2-{4-[4-(3-chloro-4-methoxybenzylamino)benzo[4,5]thieno-[2,3-d]pyrimidin-2-yl]cyclohexyl-1-yl}acetic acid;
(g) 4-[4-(3,4-methylenedioxybenzylamino)benzo[4,5]thieno[2,3-d]-pyrimidin-2-yl]cyclohexanecarboxylic acid;
(h) 4-[4-(3,4-methylenedioxybenzylamino)benzo[4,5]thieno[2,3-d]-pyrimidin-2-yl]benzoic acid;
(i) 4-[4-(3,4-methylenedioxybenzylamino)benzo[4,5]thieno[2,3-d]-pyrimidin-2-yl]phenylacetic acid;
(k) 4-[4-(3-chloro-4-methoxybenzylamino)benzo[4,5]thieno[2,3-d]-pyrimidin-2-yl]cyclohexanecarboxylic acid, ethanolamine salt;
and physiologically acceptable salts and/or solvates thereof.

## Revendications

1. Utilisation d'inhibiteurs PDE V de la formule I 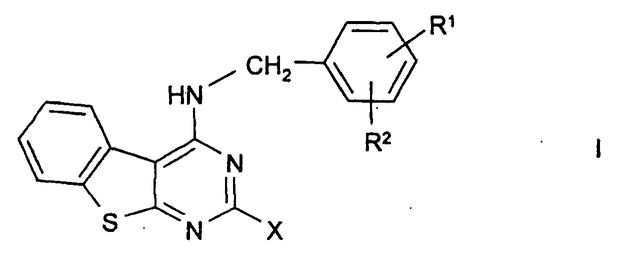 dans laquelle
R¹, R² représentent chacun indépendamment l'un de l'autre H, A, OA, OH ou Hal,
R¹ et R² également ensemble représentent alkylène comportant de 3 à 5 atomes de C, -O-CH₂-CH₂-, -CH₂-O-CH₂-, -O-CH₂-O- ou -O-CH₂-CH₂-O-,
X représente R⁴, R⁵ ou R⁶ monosubstitué par R⁷,
R⁴ représente alkylène linéaire ou ramifié comportant de 1 à 10 atomes de C, où un ou deux groupes CH₂ peuvent être remplacés par des groupes -CH=CH-,
R⁵ représente cycloalkyle ou cycloalkylalkylène comportant de 5 à 12 atomes de C,
R⁶ représente phényle ou phénylméthyle,
R⁷ représente COOH, COOA, CONH₂, CONHA, CON(A)₂ ou CN,
A représente alkyle comportant de 1 à 6 atomes de C et
Hal représente F, CI, Br ou I,
et leurs sels physiologiquement acceptables et/ou leurs solvats, pour la préparation d'un médicament pour le traitement d'un dysfonctionnement de l'érection chez les hommes qui sont simultanément traités à l'aide de vasodilatateurs qui agissent via le système NO-cGMP.

2. Utilisation selon la revendication 1, où les vasodilatateurs sont choisis parmi le groupe des composés de nitrate.

3. Utilisation selon l'une des revendications précédentes, où l'inhibiteur PDE V est choisi parmi le groupe
(a) acide 3-[4-(3-chloro-4-méthoxybenzylamino)benzo[4,5]thiéno-[2,3-d]pyrimidine-2-yl]propionique;
(b) acide 4-[4-(3,4-méthylènedioxybenzylamino)benzo[4,5]thiéno-[2,3-d]pyrimidine-2-yl]butyrique;
(c) acide 7-[4-(3,4-méthylènedioxybenzylamino)benzo[4,5]thiéno-[2,3-d]pyrimidine-2-yl]heptanoïque;
(d) acide 7-[4-(3-chloro-4-méthoxybenzylamino)benzo[4,5]thiéno-[2,3-d]pyrimidine-2-yl]heptanoïque;
(e) acide 5-[4-(3-chloro-4-méthoxybenzylamino)benzo[4,5]thiéno-[2,3-d]pyrimidine-2-yl]valérique;
(f) acide 2-{4-[4-(3-chloro-4-méthoxybenzylamino)benzo[4,5]thiéno-[2,3-d]pyrimidine-2-yl]cyclohexyl-1-yl}acétique;
(g) acide 4-[4-(3,4-méthylènedioxybenzylamino)benzo[4,5]thiéno-[2,3-d]pyrimidine-2-yl]cyclohexanecarboxylique;
(h) acide 4-[4-(3,4-méthylènedioxybenzylamino)benzo[4,5]thiéno-[2,3-d]pyrimidine-2-yl]benzoïque;
(i) acide 4-[4-(3,4-méthylènedioxybenzylamino)benzo[4,5]thiéno-[2,3-d]pyrimidine-2-yl]phénylacétique;
(k) acide 4-[4-(3-chloro-4-méthoxybenzylamino)benzo[4,5]thiéno-[2,3-d]pyrimidine-2-yl]cyclohexanecarboxylique, sel d'éthanol-amine;
et leurs sels physiologiquement acceptables et/ou leurs solvats.
